# EUROPEAN PATENT APPLICATION

(11) **EP 3 945 201 A1**
(43) Date of publication of application: **02.02.2022**
(21) Application number: 20000275.6
(22) Date of filing: 01.08.2020
(51) Int. Cl.: E21B 47/00, G01F 1/66, G01N 29/02, G01N 33/28, G01N 27/22

(54) **CONTROLLING OF AN OIL WELL BASED ON FLUID PARAMETERS**

(71) Applicant: Otto-von-Guericke-Universität Magdeburg, 39106 Magdeburg (DE)
(72) Inventor: MUKHIN, Nikolay, 39106 Magdeburg (DE); KUTIA, Mykhailo, 39114 Magdeburg (DE); AMAN, Alexander, 39110 Magdeburg (DE)

(57) **Abstract**

The invention relates to a sensor device (2) for measuring at least one parameter of an fluid (F) flowing inside an oil well, comprising: a tubing (16) which is connectable at both ends (24, 26) to parts (12, 14) of the tubing (10) of the oil well (1) with an inner cavity (28), at least one dielectric sensor (30) arranged in a recess (32) that is integrated into an inner surface (34) of the tubing, at least one acoustic sensor (38) arranged at an outer surface (36) of the tubing, and a control and transmission unit (44) for controlling the at least one acoustic sensor and the at least one dielectric sensor and for transmitting sensor data to a surface unit (46). Furthermore, the invention relates to a sensor system, a method for determining at least one parameter of a fluid flowing through a tubing and a method for controlling of an oil well based on at least one determined parameter of a fluid flowing through a tubing.

## Description

The disclosure relates to a sensor device for measuring at least one parameter of a fluid flowing inside a tubing of an oil well. Furthermore, the disclosure relates to a sensor system, a method for determining at least one parameter of a fluid flowing through a tubing of an oil well and to a method for controlling an oil well.

During the production of oil multiple solid, fluid and gaseous elements may reach the surface. Depending on these elements inside the fluid flowing in the tubing of an oil well a risk of hazard may occur. Thus, it is essential to determine the physical and chemical properties of the fluid flowing in the tubing of an oil well in order to provide safe operation of the oil well and to control the production of the oil well.

Different methods for monitoring the characteristics of the fluid inside the tubing of the oil well are known. For example sensors may be arranged at protrusion openings at walls of the tubing of the oil well. Further methods arrange sensors inside the tubing of the oil well. Thus, the known methods either violate the integrity of the tubing by protrusions or by placing sensors in the annular space or the hydraulic parameters of the inner part of the tubing are changed due to the presence of sensor placed inside the tubing and interacting with the fluid flow.

External sensor arrangements which are attached at an outer surface of the tubing of the oil well require modifications of the tubing. Thus, the design of the tubing is more complicate and prone to failure.

Furthermore, methods for optical analysis of the fluid inside the tubing of the oil well are known. Such methods require optical windows inside the wall of the tubing, thus reducing the structural integrity of the tubing.

An objective of the present disclosure is to provide a sensor device and a sensor system which allow an improved controlling an oil well without affecting the structural integrity of the components of the oil well and the fluid flow. This object is achieved by the subject-matter of the independent claims. Further developments of the subject-matter of the independent claims are provided in the sub-claims.

According to one aspect of the present disclosure a sensor device for measuring at least one parameter of a fluid flowing inside an oil well is provided.

The sensor device comprises a tubing which is connectable at both ends to parts of the oil well tubing such that an inner cavity of the tubing is connected with inner cavities of the parts of the oil well tubing. Preferably, the inner cavity of the tubing has the same dimension of its cross section as the inner cavities of the parts of the oil well tubing. Thus, the fluid flow is not affected by the tubing of the sensor device.

The oil well comprises a casing that structurally reinforces a bore hole and reaches at least until an oil bearing zone or the production zone. The casing comprises perforations in order to allow oil ingress into the casing of the oil well. Inside the casing the oil well tubing is installed. The tubing of the oil well is a pipe through which the oil inside the casing of the oil well may flow to the surface.

Since the oil is only one of many gaseous, solid and fluid components produced by the oil well, in the following description the produced medium is defined as fluid.

Depending on the characteristics of the oil well, the fluid may flow through the tubing of the oil well by its own pressure or by applying external forces, for example with pumps, pumpjacks or by introduction of fluids into the oil well.

The parts of the oil well may comprise pipes or tubes of the oil well, filters of the oil well and/or well heads of the oil well. Depending on the depth of the oil well the tubing of the oil well may comprise multiple tubing sections connected with each other. Thus, the tubing of the sensor device for example may be inserted between two such tubing sections or segments of the oil well. Thus, the initial construction of the oil well stays unmodified.

Furthermore, the sensor device comprises at least one dielectric sensor arranged in a recess which is integrated into an inner surface of the tubing. Preferably, the dielectric sensor is not protruding into the flow of the fluid through the tubing of the sensor device. Thus, any interaction of the dielectric sensor with the fluid flow is avoided.

The sensor device comprises at least one acoustic sensor arranged at an outer surface of the tubing. By using sensor data of the acoustic sensor and the dielectric sensor, dielectric constant of the fluid, the frequency and quality factor of the appearance of acoustic resonance peaks at various acoustic modes may be determined, which are sensitive to density, speed of sound, and fluid viscosity. Based on these relations, the at least one dielectric sensor and the at least one acoustic sensor are capable to monitor changes in the phase composition, component composition and fluid characteristics depending on pressure and temperature (viscosity, density and sound velocity) simultaneously, while the fluid moves through the tubing of the sensor device.

Moreover, the sensor device comprises a control and transmission unit for controlling the at least one acoustic sensor and the at least one dielectric sensor and for transmitting sensor data from the at least one acoustic sensor and the at least one dielectric sensor to a surface unit.

According to an embodiment, the sensor device integrates all necessary sensors into the tubing, the electronics and the power system of the control and transmission unit is mounted externally e.g. outside the inner cavity of the tubing. The form factor for mounting electronic components may be adjusted depending on the process, the well casing and the used tubings of the oil well.

By using the sensor device, a plurality of sensor data of various characteristics of the fluid flowing through the tubing of the sensor device may be obtained and used for detailed analysis of the fluid produced by the oil well.

According to one embodiment the at least one acoustic sensor comprises active acoustic elements and passive acoustic elements. The active acoustic elements are configured for generating acoustic waves and for receiving reflected acoustic waves. The passive acoustic elements are configured to reflect acoustic waves. The tubing of the sensor device may be inserted between two existing tubings or parts of tubings of the oil well. The tubing of the sensor device may be formed as a cylindrical acoustic resonator of the same inner diameter as the pipeline of the oil well. The passive acoustic elements may be formed at the outer surface of the tubing.

According to an alternative embodiment, the passive acoustic elements may be integrally formed with the tubing of the oil well. Thus, the tubing and the passive acoustic elements may be a one piece component.

Preferably, the passive acoustic elements of the acoustic sensor may comprise a form and/or thickness which results in a reflection of acoustic waves generated by the active acoustic elements of the acoustic sensor.

For example, the passive acoustic elements may comprise a rectangular, triangular, circular or a ring form.

According to another embodiment the passive acoustic elements may be made of tubing material or may comprise a different material composition as the tubing material of the sensor device.

Furthermore, the passive acoustic elements may be arranged as a symmetrical or asymmetrical structure on the outer surface of the tubing of the sensor device. The active acoustic elements may be integrated into the structure of the passive acoustic elements. For example, the passive acoustic elements and active acoustic elements may be arranged in an alternating manner, so that one active acoustic element is surrounded by at least one passive acoustic element.

According to another example, one of the plurality of active acoustic elements may be arranged adjacent to at least one passive acoustic element.

The individual active acoustic elements are made of piezoceramics for example and are electrically connected to the control and transmission unit. Thus, the control and transmission unit may control the active acoustic elements in order to generate acoustic waves. Furthermore, the active acoustic elements may receive the reflected acoustic waves and convert the acoustic oscillations into electrical signals which are received and evaluated by the control and transmission unit. Depending on the configuration, the control and transmission unit may transmit the raw electrical signals of the at least one active acoustic element to a surface unit without processing or evaluation.

In one further embodiment the passive acoustic elements are made from the same material as the tubing. Thus, the interaction of the generated acoustic waves may be precisely predicted and planned prior to the use of the sensor device. The shape and the arrangement of the passive acoustic elements may be adjusted according to configuration, size and accuracy of the sensor device.

The number of active acoustic elements and passive acoustic elements may vary depending on the required accuracy, sensitivity and information content of the measurements of the sensor device. The higher the requirements, the more acoustic sensors with passive acoustic elements and active acoustic elements are required.

According to a further embodiment the active acoustic elements in a central section of the tubing are configured for generating acoustic waves and for receiving reflected acoustic waves, wherein the central section is arranged between an inlet section and an outlet section of the tubing of the sensor device. Thus, the active acoustic elements in the central section of the sensor structure or sensor device are designed to generate acoustic fields and detect the response of the generated acoustic fields.

The inlet section of the tubing may comprise a connection in order to connect the sensor device to one part of the tubing of the oil well. The outlet section may also comprise a connection in order to connect the sensor device to a second part of the tubing of the oil well.

Depending on the requirements of the sensor device, the central section may comprise the same size as the inlet section and the outlet section. The central section, the inlet section and the outlet section may be formed cylindrically and may divide the tubing of the sensor device into three parts or sections across the flowing direction of the fluid.

According to one embodiment, the active acoustic elements and/or the passive acoustic elements arranged in the outlet section and in the inlet section of the tubing are configured to focus acoustic waves to the central section of the tubing. The passive acoustic elements on the sides or inlet section or outlet section partially reflect the acoustic waves propagating along the tubing of the sensor device, which creates the effect of focusing the acoustic waves to the central section of the sensor device.

In order to increase the accuracy, sensitivity and information content of measurements, the number of active piezoelectric elements in the central part of the sensor device can be increased. Additionally, the active and passive elements on the inlet and outlet sections at the outer surface of the tubing can be arranged. For example one circumferential row of active acoustic elements or passive acoustic elements on the outer surface of the tubing may be increased to two or more rows of active acoustic elements or passive acoustic elements.

Accordingly, the number of dielectric sensors at the inner surface of the tubing of the sensor device may vary depending on the requirements. The dielectric sensors may be constructed as capacitor cells and may be arranged in circled along an inner circumference of the inner cavity,

The acoustic sensors and dielectric sensors are integrated into the pipeline or tubing of the oil well by replacing a portion of the tubing of the oil well with the tubing of the sensor device with the sensor structure of acoustic sensors and dielectric sensors that performs acoustic and dielectric measurements of the fluid.

In one embodiment, the tubing of the sensor device is connectable by thread joint, flange joint and/or bayonet joint with parts of the tubing of the oil well. Thus, the sensor device may be integrated into the tubing of the oil well by using different connection methods. This measure prevents any impairment of the tubing of the oil well. Furthermore, the conventional equipment of the oil well tubing may be used without modifications.

According to a further aspect of the present disclosure a sensor system is provided. The sensor system comprises at least one inventive sensor device. Moreover, the sensor system comprises a surface unit and a transmission link connecting a control and transmission unit of the at least one sensor device with the surface unit. Preferably the at least one sensor device is arranged between two parts of a tubing of an oil well or at an oil well head.

The proposed sensor system provides comprehensive monitoring, control and management of both individual oil wells and whole hydrocarbon production fields with plurality of oil wells.

Especially, one sensor system may comprise multiple sensor devices arranged at one oil well or at different oil wells. Thus, one oil well may be observed by one or multiple sensor devices. Multiple sensor devices may be linked to one sensor system and may be controlled by one sensor system.

The at least one sensor device comprises a set of sensors built into its tubing. This sensor device may be integrated into the tubing of the oil well without impairing the flow of the fluid to the surface. Preferably, the control and transmission unit of the sensor device is arranged underground at the tubing of the sensor device.

According to an embodiment, the sensor device may be arranged at the bottom end of the tubing of the oil well or at the oil well head. Thus sensor data may be obtained at the entrance of the fluid into the tubing of the oil well.

The control and transmission unit of the sensor device is connected with the surface unit via the transmission link in order to transmit sensor data of the sensor device. The sensor data may be transmitted as analog electrical signals or as digital electrical signals.

The surface unit may act as an information processing unit for analyzing the sensor data and to control different control modules of the oil well like valves and pumps. Thus, the surface unit may decide based on the received sensor data how to control the oil well and the valves of the oil well.

Furthermore, the surface unit may be connected to a system for accurate short-term prediction of water, hydrocarbon inflows, phase composition changes, density changes and viscosity changes in the produced fluid.

The components of the sensor system are preferably modular. Thus, the number of used sensor devices and surface units may vary depending on the requirements.

In one embodiment the transmission link is configured as optical fiber transmission, as an electrical data transmission or as a wireless data transmission. This measure results in high flexibility of the possible transmission technologies. Furthermore, the utilized transmission technology may be dependent on the depth of the sensor device and the control and transmission unit of the sensor device.

For example, if the sensor device is near the surface wireless data transmission, like WLAN, may be used for transmission of sensor data. If the distance between the surface unit and the control and transmission unit increases, other transmission technologies like insulated electrical cables or optical fibers may be used in order to transmit the sensor data.

Preferably, two transmission links of the same or different transmission technology may be used in order to provide redundancy.

According to a further embodiment, the sensor system comprises multiple sensor devices positioned at different depths of the oil well. Thus, the parameters of the fluid can be controlled at the bottom of the oil well, along the wellbore and at the well head. This measure allows monitoring of the fluid behavior at the complete working section of the oil well.

According to a further aspect of the present disclosure a method for determining at least one parameter of a fluid flowing through a tubing of the oil well is provided. Preferably, the method may be performed by the inventive sensor system.

In one step at least one dielectric sensor is controlled to generate an alternating electric field and/or at least one acoustic sensor is controlled to generate acoustic waves for interacting with the fluid. In a further step sensor data of the dielectric sensor and/or acoustic sensor as a response to the interaction of the fluid is received. These steps may be performed by sensors of at least one sensor device of the sensor system.

After receiving the response the sensor data may be pre-processed by the control and transmission unit of the sensor device and transmitted to at least one surface unit of the sensor system.

Furthermore, the surface unit is able to control the control and transmission unit of the sensor device. Thus, the start and the end of measurements of the sensor data may be defined by the sensor system. Furthermore, a measurement frequency is adjustable by the surface unit. These features may be realized by sending control signals from the surface unit via transmission link to the control and transmission unit of the sensor device.

The received sensor data is processed by the surface unit of the sensor system and the at least one parameter of the fluid is determined in a further step.

Thus, by using the method multi-parameter on-line monitoring of a fluid inside an oil well may be provided. The fluid is transferred through the tubing of the sensor device with integrated sensors. In a simultaneous generation of acoustic waves and alternating low voltage electric fields at a section of a pipeline or the tubing od the sensor device sensor data may be obtained. Thus, spectral characteristics as result of passage of acoustic and electromagnetic waves through the analyzed fluid are measured.

According to a further embodiment, the spectral characteristics are processed by the surface unit to obtain information about at least one parameter of the flowing liquid. Especially, a viscosity of the fluid, a density of the fluid, distribution of liquid phases in the fluid, distribution of gaseous phases in the fluid, presence and amount of solid phases in the fluid and/or changes in chemical composition of the fluid are determined as the at least one parameter of the fluid based on the processing of the received sensor data.

Additionally, using on-line pressure and temperature measurements may be provided in order to obtain further parameter of the fluid. Thus, additional pressure sensors and temperature sensors may be installed in the tubing of the sensor device.

According to a further aspect of the present disclosure a method for controlling of an oil well based on at least one determined parameter of a fluid flowing through a tubing is provided. The at least one parameter may be determined by the inventive method for determining at least one parameter of a fluid flowing through a tubing.

In one step, the at least one parameter of the fluid is received. Based on the received at least one parameter of the fluid a production of the oil well is stopped or an operating mode of the oil well is changed. Furthermore, the operation mode of the oil well may stay unchanged based on the at least received parameter. Thus, the oil well may be directly controlled by the surface unit of the sensor system as processing unit.

Preferably, the at least one parameter is obtained by information processing of the sensor system located on the surface of acoustic and dielectric spectra from an underground measurement system or sensor device and its analysis to obtain data on fluid properties.

By utilizing the method, a possibility of urgent transmission of signals to the mechanical control system of the oil well to stop production or change the operating mode may be generated.

The method for controlling the operation of an oil well may be provided to the oil well that is in a flow mode and in a transient mode of operation. Monitoring may be carried out by a group of sensors of the sensor device integrated into a tubing, which do not create additional local resistances to the fluid. Based on the sensor data of these sensors monitoring of changes in the phase composition, component composition and fluid characteristics depending on pressure and temperature is possible, while the fluid moves through the tubing of the oil well.

Thus, a fast and automatic respond on unforeseen difficulties by a signal to shut down the oil well valves is realized.

Using the method, the dielectric constant of the fluid becomes determined, as well as the frequency and quality factor of the appearance of acoustic resonance peaks at various acoustic modes, which are sensitive to density, speed of sound, and fluid viscosity.

To calculate the quality factor of the peaks of acoustic resonances, a preliminary calculation and simulation of the sensor are performed, with the selection of the optimal geometry of the outer part of the pipeline.

Information from the sensors or the sensor data is fed through the transmission link to the surface unit, where the sensor data is for example processed, interpreted and are compared with predetermined boundary conditions. Depending on predefined rules a signal is generated and sent from the surface unit to at least one control device of the oil well that can either block or change at least one diaphragm value to control a throughput of at least one valve of the oil well. In this way the flow rate and depression on the reservoir can be controlled by generated control signals of the surface unit based on the interpreted sensor data.

In one further embodiment, the received, at least one parameter of the fluid is utilized as input of a curve, a look-up table, a calculation model and/or a neuronal network, wherein control signals for controlling the oil well are generated based on an output of the curve, the look-up table, the calculation model and/or the neuronal network. Thus, different dynamic and static methods may be used in order to provide control signals for controlling valves and/or operation modes of the oil well.

Additional data, for example of threshold values, can be obtained from several sources like data on a study of the oil well and oil well field, data from a project for a development of an oil well field or from analysis of data cumulatively accumulated during the operation of the sensor device with the construction of trend dependencies.

Furthermore, a creation of a full-fledged field model with automatic updates by receiving new sensor data from the sensor system may be provided.

The data obtained from the sensors can be used in parallel in several calculated software cores, depending on the technological requirements. The control system of the well may work based on solving a system of equations that select the best result for the fluid flow rate for a given period of time. The initial data for this system are the boundary conditions specified by the parameters of the oil well and the oil well field.

Sensor data from the sensor system may be processed in real time. Furthermore a cumulatively accumulated database and also data on forecasting changes in production parameters may be received in order to provide a control of the oil well.

According to one embodiment, based on the received at least one parameter of the fluid a short-term model of a behavior of the oil well is determined, wherein control signals for controlling the oil well are generated based on outputs of the short-term model. Thus, a system of a short-term model, for example within a week, of the behavior of the fluid in the tubing of the oil well and continuous automatic adjustment by obtaining data or parameters on the current properties of the fluid, as well as transmitting signals to stop production or change the operating mode of the oil well may be created or operated. By this measure crashes or malfunctions during the oil well operation may be prevented.

Furthermore, according to another embodiment, the received at least one parameter of the fluid and/or the short-term model of the behavior of the oil well are transmitted to a long-term field modeling system. Thus, automatic data transfer to long-term field modeling systems to adjust the technological and economic performance of the field is provided.

The present disclosure will be explained in more detail below on the basis of schematic embodiments shown in the accompanying drawings:
- Fig. 1: shows a sectional view of an oil well to illustrate a build in position of a sensor device according to an embodiment of the invention,
- Fig. 2: shows a perspective view of the sensor device introduces in Fig. 1,
- Fig. 3: shows a schematic structure of sensor system according to an embodiment of the invention, and
- Fig. 4: illustrates a method for controlling of an oil well based on at least one determined parameter of a fluid flowing through a tubing of the sensor device.

In the figures, identical or corresponding elements are marked with the same reference numerals.

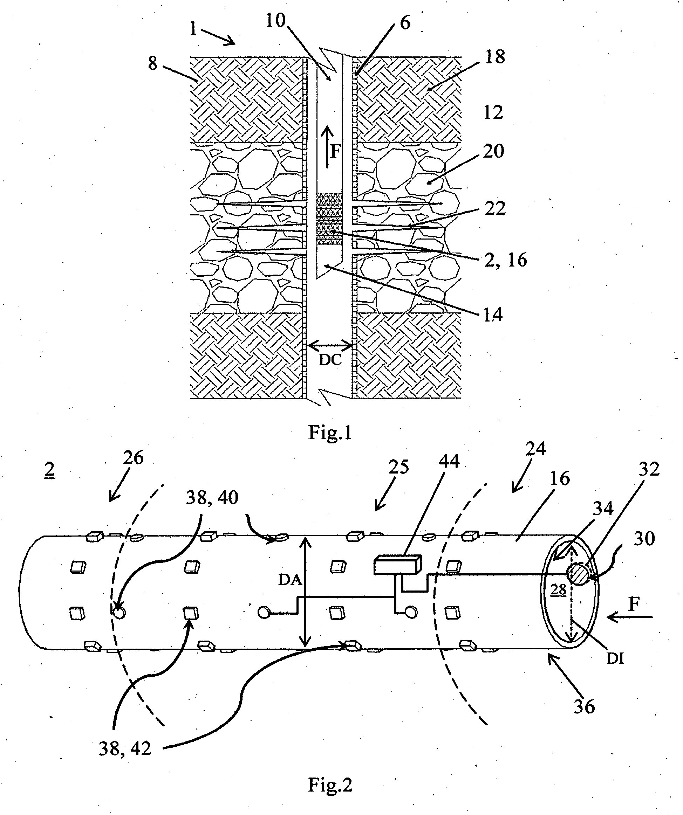

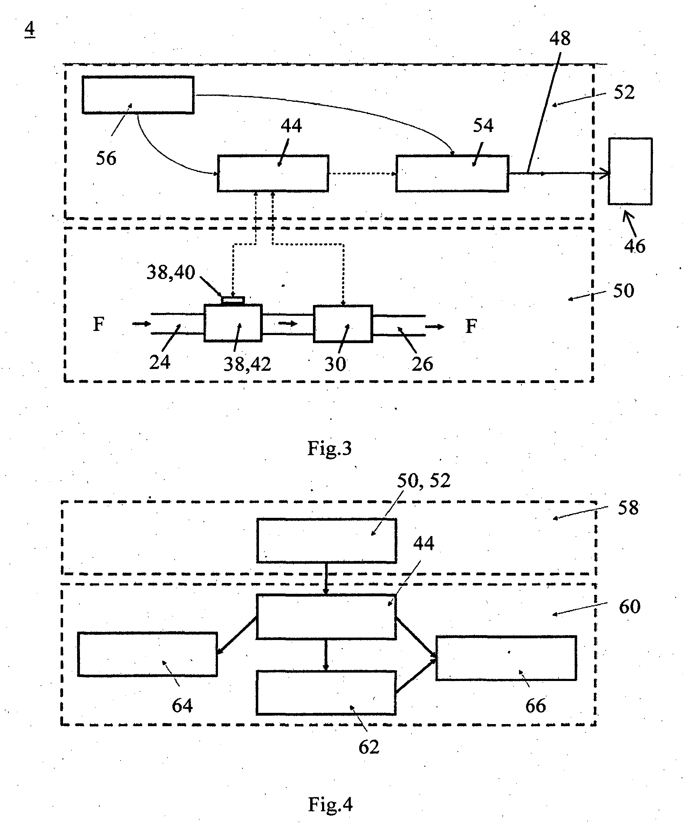

Fig. 1 shows a sectional view of an oil well 1 to illustrate a build in position of a sensor device 2 according to an embodiment of the invention. A sensor system 4, which is described in Fig. 3, may comprise multiple sensor devices 2, but for overview purposes only one sensor device 2 is described.

The oil well 1 comprises a casing 6 that enforces a bore hole inside a ground 8. In the casing 6 a tubing 10 of the oil well 1 is positioned in order to produce a fluid F by transferring the fluid F to a surface.

The tubing 10 of the oil well 1 comprises at least two parts 12, 14. In the shown example, the tubing 10 of the oil well 1 comprises a pipe 12 of the oil well 1 and a well head 14.

The sensor device 2 is installed under the surface of the ground 8 and comprises a tubing 16. The tubing 16 of the sensor device 2 is attached between the pipe 12 of the oil well 1 and the well head 14 of the oil well 1. Thus, the fluid F flowing from the oil well 1 to the surface 36 flows through the sensor device 2.

For the optimized accuracy of the measurements, the sensor device 2 is installed below a cover 18 of a productive horizon 20 at perforations 22 penetrated inside the productive horizon 20, so that a hydrostatic pressure inside the sensor device 2 coincides with the pressure at the bottom of the oil well 1.

Preferably, the outer diameter DA of the sensor device 2 is less than a diameter DC of the production casing 6 of the oil well 1.

Fig. 2 shows a perspective view of the sensor device introduces in Fig. 1. The sensor device 2 is configured for measuring at least one parameter of the fluid F flowing inside the tubing 10 of the oil well 1.

The sensor device 2 comprises a tubing 16 which is connectable at both ends 24, 26 to parts 12, 14 of the tubing 10 of the oil well 1. By providing such connection the fluid F may flow through an inner cavity 28 of the tubing 16 of the sensor device 2. A flange joint or a bayonet joint may be used for such an integration of the tubing 16 of the sensor device 2 into the tubing 10 of the oil well 1.

The tubing 16 comprises an inlet section 24 and an outlet section 26 as a first end 24 and a second end 26. Between the inlet section 24 and the outlet section 26 a central section 25 of the tubing 16 is arranged.

Furthermore, the sensor device 2 comprises at least one dielectric sensor 30 arranged in a recess 32 that is integrated into an inner surface 34 of the tubing 16. The dielectric sensor 30 is built into the inside of the tubing 16. A recess 32 is milled on the inner surface 34 to insert a not shown ceramic touch pad. The ceramic touch pad is a ceramic base on which the mask of electrodes forming the capacitor system is applied.

A plurality of acoustic sensors 38 are arranged at an outer surface 36 of the tubing 16. The acoustic sensors 38 comprise active acoustic elements 40 and/or passive acoustic elements 42. The active acoustic elements 40 are constructed as piezoceramics.

The active acoustic elements 40 are configured for generating acoustic waves and for receiving reflected acoustic waves. The passive acoustic elements 42 are configured to reflect acoustic waves into the inner cavity 28. The passive acoustic elements 42 are made from the same material as the tubing 16.

All active acoustic elements 40 work both in phase and with phase shift at resonant frequencies of the inner cavity 28, creating several options for the distribution of the acoustic field inside the tubing 16. Thus, different portions of the fluid F in the tubing may be analyzed. For example peripheral sections, when the maximum intensity of the acoustic field is created near the tubing 16 interfaces 24, 26, as well as at the central section 25, when the maximum intensity of the acoustic field is created near the rotational axis of the tubing 16, and intermediate options may be analyzed.

Furthermore, the sensor device 2 comprises a control and transmission unit 44 for controlling the at least one acoustic sensor 38 and the at least one dielectric sensor 30. The control and transmission unit is configured to transmit sensor data of the sensors 30, 38 to a surface unit 46 of the sensor system 4.

Since the dielectric sensor 30 does not protrude into the inner cavity 28 an inner diameter DI of the tubing 16 may have the same dimension as an inner diameter of the parts 12, 14 of the oil well 1 tubing 10. Thus, the fluid F flow is not affected by the sensor device 2. Furthermore, when becomes integrated into the tubing 10 of the oil well 1, the sensor device 2 does not imply protrusions, windows, holes sty and local changes in the internal diameter of the tubing.

The active acoustic elements 40, the passive acoustic elements 42 and the control and transmission unit 44 are arranged at the outer surface 36 of the tubing 16. Preferably, the active acoustic elements 40, the passive acoustic elements 42 and the control and transmission unit 44 are located around the pipe or tubing 16 of the sensor device 2.

The control and transfer unit 44 is electrically connected with the dielectric sensor 30 through a cable connection which protrudes through the wall of the tubing 16 to the recess 32.

A simple configuration of the sensor device 2 may comprise one capacitor cell or dielectric sensor 30 on the inner surface 34 of the tubing 16, one ring of three or more piezoelectric elements or active acoustic elements 38 located equidistantly on the outer surface 36 of the tubing 16, located between passive elements 42 which are arranged as rings around the outer surface 36 of the tubing 16. The configuration is not limited to one ring of piezoelectric elements. Furthermore, an arrangement of multiple rings each with at least three piezoelectric elements is possible. The number of utilized active acoustic elements 38, the passive acoustic elements 40 and the dielectric sensors 30 may be increased in order to improve the accuracy of the sensor device 2.

Fig. 3 shows a schematic structure of sensor system 4 according to an embodiment of the invention. The sensor system 4 comprises at least one sensor device 2 and a surface unit 46. A transmission link 48 is configured for connecting the control and transmission unit 44 of the sensor device 2 with the surface unit 46.

The sensor system 4 consists of components 50 integrated into the tubing 16 and components 52 located at the outer surface 36 of the tubing 16 between the casing 6 of the oil well 1 and the tubing 16 of the sensor device 2.

The fluid F passes through the tubing 16 from the inlet section 24, the central section 25 and the outlet section 26. At the tubing 16, the acoustic sensors 38 and the dielectric sensors 30 are located.

The control and transmission unit 44 provides signals to the active acoustic elements 40 of the acoustic sensors 38 which act as acoustic signal generator-receiver.

Furthermore, the control and transmission unit 44 provides signals to the dielectric sensors 30 and receives acoustic and dielectric spectra. The control and transmission unit 44 transmits information to a surface unit 46 of the sensor system 4 by optionally using an external communication module 54.

The control and transmission unit 44 and the optional communication module 54 are powered by a power source 56. The power source 56 is for example a low voltage battery.

The transmission link 48 to the surface unit 46 may be a digital signal transmission based on electrical signals or optical signals.

The surface unit 46 is for example constructed as a signal processing system consisting of a computer with appropriated software that interprets the received data from downhole sensors 30, 38 and allows adjusting oil field models that provides correct modes of operation of wells 1 and fields, and determines in real time necessary fluid parameters of the fluid F.

Fig. 4 illustrates a method for controlling of an oil well 1 based on at least one determined parameter of a fluid F flowing through a tubing 16 of the sensor device 2.

The entire oil well operation monitoring or sensor system 4 may be divided into underground components 58 and overground components 60 which are described in Fig. 3.

The surface unit 44 is located on the surface and may transmit data to a short-term modeling 62 and long-term well/field modeling 64. Furthermore, the surface unit 44 can generate control signals for controlling 66 the oil well 1 based on outputs of the short-term model 62 or directly based on sensor data.

### Reference signs

- 1: oil well
- 2: sensor device
- 4: sensor system
- 6: casing of the oil well
- 8: ground
- 10: tubing of the oil well
- 12: first part of the oil well / pipe section of the oil well
- 14: second part of the oil well / well head
- 16: tubing of the sensor device
- 18: cover of the productive horizon
- 20: productive horizon
- 22: perforations penetrated inside the productive horizon
- 24: first end of tubing / inlet section
- 25: central section
- 26: second end of tubing / outlet section
- 28: inner cavity
- 30: dielectric sensor
- 32: recess for positioning the dielectric sensor
- 34: inner surface of the tubing
- 36: outer surface of the tubing
- 38: acoustic sensor
- 40: active acoustic element
- 42: passive acoustic element
- 44: control and transmission unit
- 46: surface unit
- 48: transmission link
- 50: components integrated into the tubing
- 52: components at the outer surface of the tubing
- 54: communication module
- 56: Power source
- 58: underground components
- 60: overground / surface components
- 62: short-term modelling system
- 64: long-term modelling system
- 66: oil well control

- DA: outer diameter of the tubing of the sensor device
- DI: diameter of the inner cavity
- DC: inner diameter of the casing of the oil well
- F: Fluid

## Claims

1. Sensor device (2) for measuring at least one parameter of a fluid (F) flowing inside a tubing (10) of an oil well (1), comprising:
- a tubing (16) which is connectable at both ends (24, 26) to parts (12, 14) of the tubing (10) of the oil well (1) with an inner cavity (28),
- at least one dielectric sensor (30) arranged in a recess (32) that is integrated into an inner surface (34) of the tubing (16),
- at least one acoustic sensor (38) arranged at an outer surface (36) of the tubing (16), and
- a control and transmission unit (44) for controlling the at least one acoustic sensor (38) and the at least one dielectric sensor (30) and for transmitting sensor data to a surface unit (46).

2. Sensor device according claim 1, wherein the at least one acoustic sensor (38) comprises active acoustic elements (40) and/or passive acoustic elements (42), wherein the active acoustic elements (40) are configured for generating acoustic waves and for receiving reflected acoustic waves and wherein the passive acoustic elements (42) are configured to reflect acoustic waves.

3. Sensor device according to claim 2, wherein the passive acoustic elements (42) are made from the same material as the tubing (16).

4. Sensor device according to claim 2 or 3, wherein the active acoustic elements (40) in a central section (25) of the tubing (16) are configured for generating acoustic waves and for receiving reflected acoustic waves, wherein the central section (25) is arranged between an inlet section (24) and an outlet section (26) of the tubing (16).

5. Sensor device according to anyone of the claim 2 to 4, wherein the active acoustic elements (40) and/or the passive acoustic elements (42) arranged in the outlet section (26) and in the inlet section (24) of the tubing (16) are configured to focus acoustic waves to the central section (25) of the tubing (16).

6. Sensor device according to anyone of the claim 1 to 5, wherein a diameter (DI) of the inner cavity (28) of the tubing (16) equals an inner diameter of the parts (12, 14) of the, tubing (10) of the oil well (1) and the at least one dielectric sensor (30) is arranged flush with the inner surface (34) of the tubing (16) of the sensor device (2).

7. Sensor device according to anyone of the claim 1 to 6, wherein the tubing (16) is connectable by thread joint, flange joint and/or bayonet joint with parts (12, 14) of the tubing (10) of the oil well (1).

8. Sensor system (4), comprising at least one sensor device (2) according to any of the claims 1-7, comprising a surface unit (46) and a transmission link (48) connecting a control and transmission unit (44) of the sensor device (2) with the surface unit (46), wherein the at least one sensor device (2) is arranged between two parts (12, 14) of a tubing (10) of an oil well (1) or at an oil well head (14).

9. Sensor system according to claim 8, wherein the transmission link (48) is configured as optical fiber transmission, as an electrical data transmission or as a wireless data transmission.

10. Sensor system according to claim 8 or 9, wherein the sensor system (4) comprises multiple sensor devices (2) positioned at different depths of the oil well (1).

11. Method for determining at least one parameter of a fluid (F) flowing through a tubing (10) of the oil well (1), wherein
- at least one dielectric sensor (30) is controlled by the control and transmission unit (44) to generate an alternating electric field and/or at least one acoustic sensor (38, 40) is controlled to generate acoustic waves for interacting with the fluid (F),
- sensor data of the dielectric sensor (30) and/or acoustic sensor (38, 40) as a response to the interaction of the fluid (F) is received by the control and transmission unit (44) and/or by the surface unit (46),
- the received sensor data is processed and the at least one parameter of the fluid (F) is determined.

12. Method according to claim 11, wherein a viscosity of the fluid (F), a density of the fluid (F), distribution of liquid phases in the fluid (F), distribution of gaseous phases in the fluid (F), presence and amount of solid phases in the fluid (F) and/or changes in chemical composition of the fluid (F) are determined as the at least one parameter of the fluid (F) based on the processing of the received sensor data.

13. Method for controlling of an oil well (1) based on at least one determined parameter of a fluid (F) flowing through a tubing (16) by a method according to anyone of the claims 11-12, wherein
- the at least one parameter of the fluid (F) is received,
- based on the received at least one parameter of the fluid (F) a production of the oil well (1) is stopped or an operating mode of the oil well (1) is changed by opening and closing at least one valve.

14. Method according to claim 13, wherein the received at least one parameter of the fluid (F) is utilized as input of a curve, a look-up table, a calculation model and/or a neuronal network, wherein control signals for controlling (66) the oil well (1) are generated based on an output of the curve, the look-up table, the calculation model and/or the neuronal network.

15. Method according to claim 13 or 14, wherein based on the received at least one parameter of the fluid (F) a short-term model (62) of a behavior of the oil well (1) is determined, wherein control signals for controlling (66) the oil well (1) are generated based on outputs of the short-term model (62) for controlling a production of the oil well (1), and/or
wherein the received at least one parameter of the fluid (F) and/or the short-term model (62) of the behavior of the oil well (1) are transmitted to a long-term field modeling system (64).
